# EUROPEAN PATENT APPLICATION

(11) **EP 4 597 106 A1**
(43) Date of publication of application: **06.08.2025**
(21) Application number: 23870585.9
(22) Date of filing: 21.09.2023
(51) Int. Cl.: G01N 33/543, G01N 33/569, G01N 33/53

(54) **MAGNETIC BEAD SORTING METHOD AND DEVICE AND STORAGE MEDIUM**

(30) Priority: 27.09.2022 CN 202211182777
(71) Applicant: Shenzhen Cellbri Bio-Innovation Technology Co., Ltd., Shenzhen, Guangdong 518107 (CN)
(72) Inventor: SHANG, Yuanfang, Shenzhen, Guangdong 518107 (CN); GUO, Xiaoliang, Shenzhen, Guangdong 518107 (CN); OU, Yingting, Shenzhen, Guangdong 518107 (CN); LIU, Fujing, Shenzhen, Guangdong 518107 (CN); ZHANG, Jinxin, Shenzhen, Guangdong 518107 (CN)
(74) Representative: Herrero & Asociados, S.L.
(86) International application number: PCT/CN2023/120444
(87) International publication number: WO 2024/067353

(57) **Abstract**

The present disclosure discloses a magnetic bead sorting method, device, and storage medium. The method includes: placing an empty liquid bag on a magnetic platform, inputting a liquid containing immunomagnetic cells into the liquid bag, and adsorbing the immunomagnetic cells in the liquid bag through the magnetic platform; controlling the magnetic platform to swing according to preset swing parameters to evenly distribute the immunomagnetic cells adsorbed by the magnetic platform, while allowing other substances in the liquid bag except for the adsorbed immunomagnetic cells to be suspended in the liquid; performing a waste liquid discharge operation to allow the liquid in the liquid bag, except for the adsorbed immunomagnetic cells, to flow into a waste liquid bag; performing a cleaning operation on remaining immunomagnetic cells in the liquid bag; and after injecting a resuspension into the liquid bag and removing the liquid bag containing the immunomagnetic cells, confirming that a magnetic bead sorting process is complete. The immunomagnetic cells adsorbed by the magnetic platform in the present disclosure are evenly distributed without local accumulation, which improves the utilization rate of the magnetic field and achieves efficient and stable separation and collection of the immunomagnetic cells.

## Description

### CROSS REFERENCE

The present disclosure claims priority to a Chinese patent application with application number 202211182777.5, titled "Magnetic Bead Sorting Method, Device, and Storage Medium" filed with the Chinese Patent Office on September 27, 2022, the entire content of which is incorporated herein by reference.

### TECHNICAL FIELD

The present disclosure relates to the field of magnetic bead sorting technology, and more particularly, to a magnetic bead sorting method, device, and storage medium.

### BACKGROUND

Currently, in the magnetic bead sorting technology, magnets are used to adsorb immune magnetic bead cells that have bound to the magnetic beads (some cells have surface antigens that can bind with specific antibodies connected to the magnetic beads; these cells, in an external magnetic field, can be connected to the magnetic beads through antigens, thus forming immune magnetic bead cells). Subsequently, other substances that are not adsorbed by the magnet (such as cells that cannot bind with the magnetic beads) are drawn off, thereby separating the immune magnetic bead cells. The inventor realized that in the existing technology, a combination of the microfluidic chip and the magnetic field is usually used for sorting the magnetic beads. This solution has the problem of small processing volume. Therefore, when processing the large-volume cell fluid, due to the large area of the liquid bag, the flow rate of the liquid flowing through different areas of the liquid bag is not consistent, which leads to inconsistent magnetization in the corresponding areas of the liquid bag. For example, when the flow rate in some areas is too high, the immunomagnetic cells may escape due to insufficient contact with the magnetic field. As a result, the magnet utilization rate is low and the magnetic adsorption on the immunomagnetic cells is not sufficient. Moreover, a large number of the adsorbed immunomagnetic cells may accumulate near an area with a low flow rate, which also leads to insufficient magnetic adsorption.

### SUMMARY OF THE DISCLOSURE

The present disclosure provides a magnetic bead sorting method, device, and storage medium to solve the problem of low magnetic field utilization in the existing technology.

A magnetic bead sorting method including:
placing an empty liquid bag on a magnetic platform, inputting a liquid containing immune magnetic bead cells into the liquid bag, and adsorbing the immune magnetic bead cells in the liquid bag through the magnetic platform;
controlling the magnetic platform to swing according to preset swing parameters to evenly distribute the immune magnetic bead cells adsorbed by the magnetic platform, while suspending other substances in the liquid bag except for the adsorbed immune magnetic bead cells in the liquid;
performing a waste liquid discharge operation to allow the liquid in the liquid bag, except for the adsorbed immune magnetic bead cells, to flow into a waste liquid bag;
performing a cleaning operation on remaining immune magnetic bead cells in the liquid bag;
after injecting a resuspension into the liquid bag and removing the liquid bag containing the immune magnetic bead cells, confirming that a magnetic bead sorting operation is complete.

The present disclosure further provides a controller for performing the above magnetic bead sorting method.

A magnetic bead sorting device including a pressing device, a mixing device, and the above controller for performing the magnetic bead sorting method and the controller is connected to the pressing device and the mixing device.

A computer-readable storage medium storing computer-readable instructions, wherein the computer-readable instructions, when being executed by a processor, implement the above magnetic bead sorting method.

The present disclosure can control the magnetic platform to swing according to the preset swing parameters, thereby mixing the liquid (such as cell fluid) in large-volume or high-flux liquid bags, such that the immunomagnetic cells are evenly distributed on the bottom of the liquid bag without accumulating in local positions. This allows the magnetic platform to closely adhere to the un-accumulated immunomagnetic cells in the liquid bag after mixing, achieving sufficient adsorption of the immunomagnetic cells by the magnetic platform and avoiding the loss of the immunomagnetic cells. At the same time, other substances in the liquid bag, except for the adsorbed immunomagnetic cells can be suspended in the liquid (for example, as the magnetic platform swings, other substances adhered to the already adsorbed immunomagnetic cells are shaken to detach from the immunomagnetic cells to be suspended in the liquid, facilitating the discharge of the other substances during the waste liquid discharge operation, without blocking the un-adsorbed immunomagnetic cells from being adsorbed by the magnetic platform). Furthermore, the other substances except for the immunomagnetic cells are separated through the waste liquid discharge operation and the cleaning operation, allowing for the collection of the immunomagnetic cells. The present disclosure improves the utilization efficiency of the magnetic field and achieves efficient and stable separation and collection of the immunomagnetic cells through the magnetic bead sorting process.

The details of one or more embodiments of the present disclosure are presented in the accompanying drawings and description below, and other features and advantages of the present disclosure will become apparent from the specification, drawings, and claims.

### BRIEF DESCRIPTION OF THE DRAWINGS

In order to more clearly illustrate the technical solution of the embodiments of the present disclosure, a brief introduction will be given to the accompanying drawings required for the description of the embodiments of the present disclosure. It is obvious that the accompanying drawings described below are only some embodiments of the present disclosure. For those skilled in the art, other drawings can be obtained based on these drawings without creative labor.
FIG. 1 is a flowchart of a magnetic bead sorting method according to an embodiment of the present disclosure;
FIG. 2 is a flowchart of a step S20 of the magnetic bead sorting method according to an embodiment of the present disclosure;
FIG. 3 is a partially-exploded view of a mixing device according to an embodiment of the present disclosure;
FIG. 4 is an assembly view of the mixing device according to an embodiment of the present disclosure;
FIG. 5 is an exploded view of a pressing device according to an embodiment of the present disclosure;
FIG. 6 is an assembly view of the pressing device according to an embodiment of the present disclosure; and
FIG. 7 is a schematic view of a computer device according to an embodiment of the present disclosure.

### DESCRIPTION OF REFERENCE NUMERALS

1, pressing device; 10, magnetic platform; 101, base; 1011, lifting through-hole; 1012, guide through-hole; 102, magnet block; 103, first rotating shaft; 20, lifting mechanism; 201, lifting assembly; 2011, first lifting block; 2012, second lifting block; 2013, limit plate; 2014, rotation shaft; 202, lifting rod; 203, ascending driving assembly; 2031, second motor; 2032, second driving wheel; 2033, second driven wheel; 2034, second synchronous belt; 2035, second rotating shaft; 2036, cam; 2037, mounting bearing; 2038, cam bracket; 2039, second installation hole; 2040, bearing follower; 2041, fixing plate; 2042, sensing block; 2043, photoelectric sensor; 30, covering mechanism; 301, cover plate; 302, first adsorption portion; 303, second adsorption portion; 40, pressing mechanism; 401, guide shaft; 402, spring; 403, linear bearing; 4011, stopper; 50, liquid bag; 2, mixing device; 21, first motor; 22, speed reducer; 23, first driving wheel; 24, first driven wheel; 25, first synchronous belt; 26, bearing; 27, synchronous bracket; 271, first installation hole; and 28, dual axis inclination sensor.

### PREFERRED EMBODIMENTS

Below, the technical solutions in the embodiments of the present disclosure will be clearly and completely described in conjunction with the accompanying drawings. Obviously, the described embodiments are a part of the embodiments of the present disclosure, not all of them. Based on the embodiments in the present disclosure, all other embodiments obtained by ordinary skilled persons in this field without creative labor are within the scope of protection of the present disclosure.

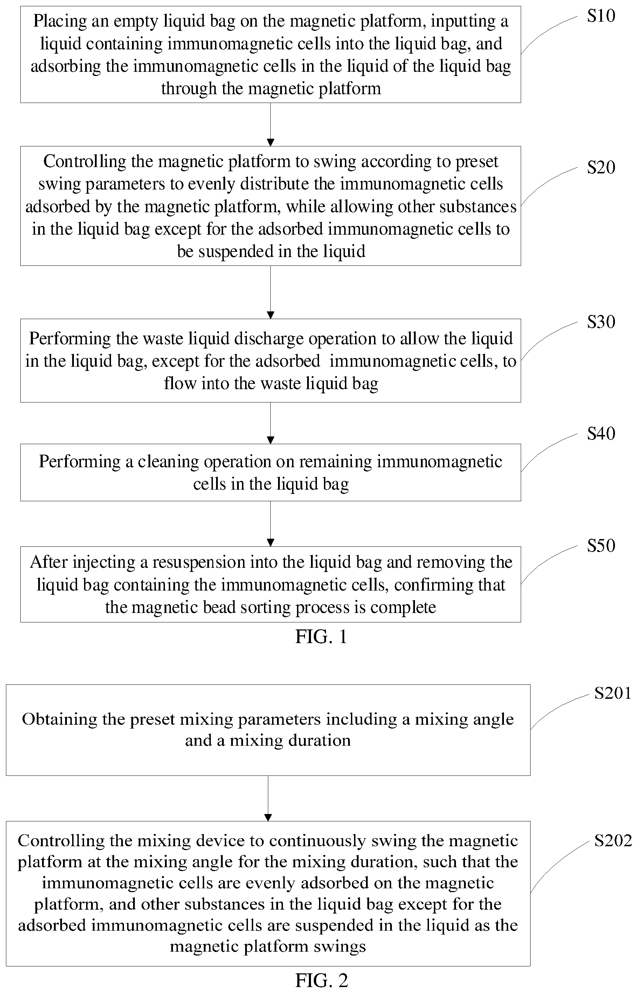

In one embodiment, as shown in FIG. 1, a magnetic bead sorting method is provided, including steps as follows.

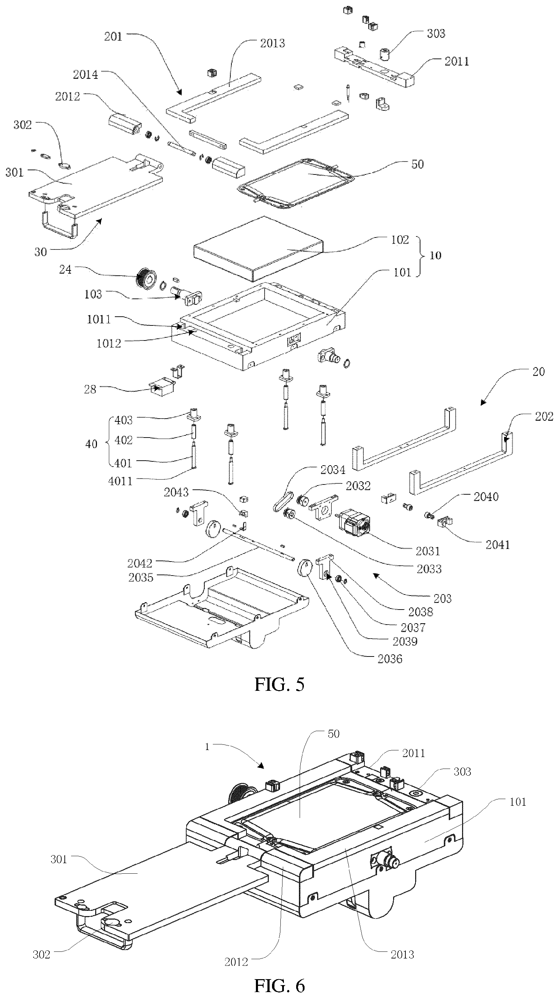

S10, placing an empty liquid bag 50 on the magnetic platform 10, inputting a liquid containing immunomagnetic cells into the liquid bag 50, and adsorbing the immunomagnetic cells in the liquid of the liquid bag 50 through the magnetic platform 10, wherein the magnetic platform 10 can be a magnetic platform formed by electromagnets or permanent magnets. For example, the magnetic platform 10 shown in FIG. 5 may include a base 101 with a lifting through-hole 1011, and a permanent magnet block 102 or an electromagnet arranged on the base 101. An installation slot is formed in the base 101 and the magnet block 102 is fixedly arranged in the installation slot. The magnetic platform 10 can be attached to a contact surface of the liquid bag 50, such that the liquid can be in close contact with a magnetic field of the magnetic platform 10, thereby adsorbing the immunomagnetic cells onto the contact surface. The liquid bag 50 is made of non-magnetic material and is not magnetically adsorbed, thus, the liquid bag 50 is not affected by the magnetic field of the magnetic platform 10.

In this step of the present disclosure, the empty liquid bag 50 is placed on the magnetic platform 10 (such as the magnet block 102), and a cell fluid (the cell fluid refers to the mixture formed by incubating cells, magnetic beads, and antibodies) in a sample container flowing into the empty liquid bag 50. That is, the sample container contains a cell fluid formed by incubating cells, magnetic beads, and antibodies, and the cell fluid contains immunomagnetic cells, which are the final target of the magnetic bead sorting method in the present disclosure. All other substances in the cell fluid, except for the immunomagnetic cells, need to be discharged into the waste liquid bag 50.

S20, controlling the magnetic platform 10 to swing according to preset swing parameters to evenly distribute the immunomagnetic cells adsorbed by the magnetic platform 10, while allowing other substances in the liquid bag 50 except for the adsorbed immunomagnetic cells to be suspended in the liquid. It can be understood that when processing the cell fluid in large volume, due to the large area of the liquid bag 50, a flow velocity of the liquid passing through each area of the liquid bag 50 is not consistent, resulting in inconsistent magnetization in each area of the liquid bag 50. Therefore, in areas with low flow rates, a large number of adsorbed immunomagnetic cells accumulate (near the areas with low flow rates), and other substances, except for the adsorbed immunomagnetic cells adhere to the already adsorbed immunomagnetic cells. Therefore, it is difficult for the area where the immunomagnetic cells (and other substances adhered to the immunomagnetic cells) accumulate (due to the accumulation of many other immunomagnetic cells and the adhered other substances between the magnetic platform 10 and the immunomagnetic cells that are still not adsorbed in the liquid, an adsorbable distance is increased and a magnetic adsorption force is weakened) to adsorb immunomagnetic cells that are still not adsorbed in the liquid, which leads to a loss of the immunomagnetic cells and a low magnetic field utilization efficiency. In this embodiment, it is necessary to avoid the accumulation of the immunomagnetic cells and the adhered other substances, thereby avoiding the loss of the immunomagnetic cells due to not being adsorbed by the magnetic platform 10. At the same time, the magnetic force of the magnetic platform 10 should not be too strong (if the magnetic force is too strong, the magnetic force may kill the cells, therefore, increasing the magnetic force cannot solve the problem of the loss of the immunomagnetic cells). Therefore, appropriate preset swing parameters are required to gently swing and mix the liquid in the liquid bag 50, such that the immunomagnetic cells no longer accumulate and are evenly distributed, and other substances in the liquid bag 50, except for the adsorbed immunomagnetic cells, no longer adhere to the adsorbed immunomagnetic cells but remain suspended in the liquid (therefore, the other substances are easy to be discharged during a waste liquid discharge operation, and may not block the un-adsorbed immunomagnetic cells from being adsorbed by the magnetic platform). At the same time, excessive swinging should be avoided (excessive swinging may cause a large number of the adsorbed immunomagnetic cells to detach from the magnetic platform 10 due to a weak magnetic force, preventing some immunomagnetic cells from being re-adsorbed and resulting in the loss of the immunomagnetic cells, and even killing the immunomagnetic cells due to the excessive swinging force). Therefore, in the present disclosure, accurate control of the preset swing parameters such as a swing angular velocity and a swing angle is required to be carried out. In some embodiments, the preset swing parameters need to be obtained first, which can include the swing angular velocity, the swing angle, a swing time, an angular acceleration, and a staying angle after the swinging of the magnetic platform 10. In an embodiment, the swing angle of the magnetic platform 10 can be within the range of ± 35 degrees (i.e. the magnetic platform 106 swings towards opposite sides, and the angle at which the magnetic platform 106 swings towards one side is within the range of 0-35 degrees; if the swing angle exceeds 35 degrees, the immunomagnetic cells may be killed). In an embodiment, the swing angle can be within the range of ± 25 degrees.

S30, performing the waste liquid discharge operation to allow the liquid in the liquid bag 50, except for the adsorbed immunomagnetic cells, to flow into the waste liquid bag 50. In this step, in order to smoothly separate and collect immunomagnetic cells, other substances in the liquid bag 50 except for the immunomagnetic cells can be discharged into the liquid bag 50 through a waste discharge pipeline by opening the waste discharge pipeline.

S40, performing a cleaning operation on remaining immunomagnetic cells in the liquid bag 50. In this step, due to the possibility of other substances adhered to the remaining immunomagnetic cells in the liquid bag 50, a cleaning pipeline can be opened to clean the adhered other substances, such that only the immunomagnetic cells are retained in the liquid bag 50 due to being adsorbed by the magnetic platform 10, thereby purifying the collected immunomagnetic cells.

S50, after injecting a resuspension into the liquid bag 50 and removing the liquid bag 50 containing the immunomagnetic cells, confirming that the magnetic bead sorting process is complete. That is, injecting the resuspension into the liquid bag 50 allows the immunomagnetic cells to float up and avoid the death of the immunomagnetic cells before culturing. Understandably, the above-mentioned resuspension can also be replaced with a liquid such as a culture medium that can maintain the activity of the immunomagnetic cells.

The present disclosure can control the magnetic platform 10 to swing according to the preset swing parameters, thereby mixing the liquid (such as the cell fluid) in the large-volume or high-flux liquid bag 50, such that the immunomagnetic cells are evenly distributed on a bottom surface of the liquid bag 50 without accumulating in local areas (such as near areas with low flow rates), thereby allowing the magnetic platform 10 to closely adhere to the immunomagnetic cells that have not accumulated in liquid bag 50 after swinging, achieving sufficient adsorption of the immunomagnetic cells by the magnetic platform 10 and avoiding the loss of the immunomagnetic cells. At the same time, other substances in the liquid bag 50, except for the adsorbed immunomagnetic cells, can be suspended in the liquid (for example, the other substances adhered to the adsorbed immunomagnetic cells are shaken to detach from the immunomagnetic cells to be suspended in the liquid, facilitating the discharge of the other substances during the waste liquid discharge operation, without blocking the still un-adsorbed immunomagnetic cells from being adsorbed by the magnetic platform 10). Furthermore, the other substances except for the immunomagnetic cells are separated through the waste liquid discharge operation and the cleaning operation, allowing for the collection of the immunomagnetic cells. The present disclosure improves the utilization efficiency of the magnetic field and achieves efficient and stable separation and collection of the immunomagnetic cells through the magnetic bead sorting process. The magnetic bead sorting process of the present disclosure can be directly performed on high-volume immunomagnetic cells, achieving a short-term high-volume magnetic bead sorting process without damaging cell viability, and there is no need for manual operation during the process, improving the efficiency of high-volume magnetic bead sorting process.

In one embodiment, the step S10 includes steps as follows.

Placing the empty liquid bag 50 on the magnetic platform 10 of a pressing device 1. As shown in FIG. 5, the pressing device 1 includes a covering mechanism 30 arranged on the magnetic platform 10 and a pressing mechanism 40 connected to the covering mechanism 30. When the covering mechanism 30 is closed, the liquid bag 50 is located in an accommodating space between the covering mechanism 30 and the magnetic platform 10. The covering mechanism 30 is arranged on the magnetic platform 10, and can close or open the above-mentioned accommodating space (when the liquid bag 50 placed in the accommodating space expands and protrudes from the accommodating space for a relatively-great height, the covering mechanism 30 cannot be closed due to being pushed up by the liquid bag 50, and forcible closing of the covering mechanism 30 may crush the liquid bag 50 or damage the immunomagnetic cells or non-nuclear immune cells inside the liquid bag 50). When not interfered by other external forces, the covering mechanism 30 can move downwards with a downward pull of the pressing mechanism 40, thereby allowing for the adjustable size of the accommodating space between the magnetic platform 10 and the covering mechanism 30 for placing the liquid bag 50.

Inputting the liquid containing immunomagnetic cells into the liquid bag 50. In the above process, a flow control assembly (not shown) can be arranged between the liquid bag 50 and the sample container, and after the flow control assembly is turned on, the liquid containing the immunomagnetic cells can be automatically input into the liquid bag 50. The liquid containing the immunomagnetic cells also can be input into the liquid bag 50 by placing the sample container at a higher position and squeezing the sample container. The flow control assembly can include a peristaltic pump that can provide power for a liquid flow, a pinch valve as a switch for the flow control assembly, and a flow regulating valve to control a flow rate of the liquid flow, or a sensor to monitor the flow rate and a pressure of the liquid flow.

After the covering mechanism 30 is closed, the pressing mechanism 40 is controlled to drive the closed covering mechanism 30 to move downwards to flatten the liquid bag 50 placed in the accommodating space between the magnetic platform 10 and the covering mechanism 30, thereby increasing the contact surface between the bottom surface of the liquid bag 50 and the magnetic platform 10, and then adsorbing the immunomagnetic cells in the liquid bag 50 onto the contact surface through the magnetic platform 10. It can be understood that after the covering mechanism 30 is closed, the closed covering mechanism 30 can be driven to move downwards by the pressing mechanism 40 and the liquid bag 50 can be evenly flattened, such that the contact surface formed by attaching the liquid bag 50 to the magnetic platform 10 is increased, and the liquid in the liquid bag 50 can be evenly distributed on the magnetic platform 10 through the contact surface and in contact with the magnetic platform 10. In this embodiment, by performing a flattening operation on the liquid bag 50, the contact surface between the bottom surface of the liquid bag 50 and the magnetic platform 10 is increased. As a result, the magnetic platform 10 can fully adsorb the immunomagnetic cells in the liquid bag 50 through the increased contact surface, avoiding the escape of the immunomagnetic cells and further improving the magnetic field utilization efficiency. Thus, efficient and stable capture of the immunomagnetic cells is achieved through a simple operation.

In one embodiment, the pressing device 1 further includes a lifting mechanism 20 connected to the covering mechanism 30. Furthermore, in step S10, after the inputting a liquid containing immunomagnetic cells into the liquid bag 50, the method further includes steps as follows.

When the covering mechanism 30 is pushed up by the liquid bag 50 which is expanded and cannot be closed, controlling the lifting mechanism 20 to drive the covering mechanism 30 to ascend, thereby increasing the accommodating space between the magnetic platform 10 and the covering mechanism 30 for placing the liquid bag 50. In this embodiment, after placing the liquid bag 50 in the accommodating space, since the liquid in the liquid bag 50 expands and protrudes from the accommodating space for a relatively-great height, the covering mechanism 30 is pushed up and opened by the liquid bag 50, thus the covering mechanism 30 cannot be closed. At this time, the lifting mechanism 20 is turned on to drive the covering mechanism 30 to ascend. As a result, the distance between the magnetic platform 10 and the covering mechanism 30 increases, and the accommodating space increases with the increased distance.

After the covering mechanism 30 ascends to a preset height at which the covering mechanism 30 can be closed, closing the covering mechanism 30. That is, when the covering mechanism 30 ascends to the preset height, the accommodating space between the covering mechanism 30 and the magnetic platform 10 is already large enough, and the covering mechanism 30 is no longer pushed up by the liquid bag 50 and can be closed normally.

Controlling the pressing mechanism 40 to drive the closed covering mechanism 30 to move downwards, thereby flattening the liquid bag 50 placed in the accommodating space, increasing the contact surface between the bottom surface of the liquid bag 50 and the magnetic platform 10, and adsorbing the immunomagnetic cells in the liquid bag 50 onto the contact surface through the magnetic platform 10. That is, after the covering mechanism 30 is closed, the closed covering mechanism 30 can be driven to move downwards by the pressing mechanism 40 and the liquid bag 50 can be evenly flattened, such that the contact surface formed by attaching the liquid bag 50 to the magnetic platform 10 increases, and the liquid in the liquid bag 50 can be evenly distributed on the magnetic platform 10 through the contact surface and in contact with the magnetic platform 10.

In one embodiment, as shown in FIGS. 5 and 6, a lifting through-hole 1011 is formed in the magnetic platform 10. The lifting mechanism 20 includes a lifting assembly 201 arranged at a top of the magnetic platform 10, a lifting rod 202 with a top thereof fixedly connected to the lifting assembly 201 by passing through the lifting through-hole 1011, and an ascending driving assembly 203 arranged below the magnetic platform 10 and connected to one end of the lifting rod 202 away from the lifting assembly 201. The covering mechanism 30 includes a cover plate 301 rotatably connected to the lifting assembly 201. A shape of the cover plate 301 can be set according to a shape of the magnetic platform 10. A shape of the lifting assembly 201 can be set according to the requirements. For example, the lifting assembly 201 can be a frame structure placed on the top of the magnetic platform 10 (or formed by multiple separated components which can move up and down simultaneously with the lifting rod 202), and can be driven by the ascending driving assembly 203 to move up and down. In one embodiment, the lifting assembly 201 includes a first lifting block 2011 arranged at a first end of the magnetic platform 10, two spaced-apart second lifting blocks 2012 arranged on a second end of the magnetic platform 10 away from the first end, a limit plate 2013 connected between the first lifting block 2011 and the corresponding second lifting block 2012, and a rotation shaft 2014 connected between the two second lifting blocks 2012. The cover plate 301 is rotatably connected to the rotation shaft 2014. In some embodiments, the lifting assembly 201 includes two of the limit plates 2013. The limit plates 2013 are arranged in parallel on opposite sides of the magnetic platform 10. In this way, the first lifting block 2011, the two second lifting blocks 2012, and the two limit plates 2013 are enclosed to form a limit frame to prevent the liquid bag 50 from escaping from the side of the accommodating space. This limit frame (which is the main component of the lifting assembly 201) can synchronously move up and down along with the cover plate 301.

Furthermore, the controlling the lifting mechanism 20 to drive the covering mechanism 30 to ascend, thereby increasing the accommodating space between the magnetic platform 10 and the covering mechanism 30 for placing the liquid bag 50 includes: turning on the ascending driving assembly 203 to drive the lifting rod 202 and thus to drive the lifting assembly 201 and the cover plate 301 to slide up along the lifting through-hole 1011 through the lifting rod 202, thereby increasing the accommodating space between the magnetic platform 10 and the cover plate 301 for placing the liquid bag 50. That is, the covering mechanism 30 is rotatably arranged on the lifting assembly 201 and moves up and down along with the lifting assembly 201. The ascending driving assembly 203 can drive the lifting assembly 201 to move up along the lifting through-hole 1011 through the lifting rod 202, which can drive the cover plate 301 to move up, thereby increasing the accommodating space between the magnetic platform 10 and the cover plate 301 for placing the liquid bag 50, and facilitating the closing of the cover plate 301.

Furthermore, as shown in FIGS. 5 and 6, the covering mechanism 30 further includes a first adsorption portion 302 arranged on the cover plate 301, and a second adsorption portion 303 arranged on the lifting assembly 201 at a position opposite to the first adsorption portion 302. The cover plate 301 is covered on the lifting assembly 201 through the adsorption between the first adsorption portion 302 and the second adsorption portion 303. At least one of the first adsorption portion 302 and the second adsorption portion 303 is a magnet; in an embodiment, both the first adsorption portion 302 and the second adsorption portion 303 can be magnets; in another embodiment, one of the first adsorption portion 302 and the second adsorption portion 303 can be a magnet and the other can be a metal with magnetic adsorption characteristics. The number, combination, specific shape and size of the first adsorption portion 302 and the second adsorption portion 303 can be set according to the needs, which are not limited here. The magnet in the present disclosure can also refer to an electromagnet. In an embodiment, the second adsorption portion 303 is an electromagnet, and the first adsorption portion 302 is an iron block. The electromagnet of the second adsorption portion 303 can be powered on to remove the magnetism of the second adsorption portion 303. At this time, magnetic adsorption does not exist between the electromagnet of the second adsorption portion 303 and the first adsorption portion 302 (iron block) on the cover plate 301, thus, the first adsorption portion 302 and the second adsorption portion 303 are in a separated state. In this way, the cover plate 301 can be opened. After the second adsorption portion 303 is powered off, the electromagnet acting as the second adsorption portion 303 generates magnetism. At this time, in the absence of external force, when the iron block on the cover plate 301 is within the magnetic adsorption range of the electromagnet, the first adsorption portion 302 of the cover plate 301 is adsorbed by the second adsorption portion 303, thus completing the closing of the cover plate 301.

In one embodiment, as shown in FIGS. 5 and 6, the ascending driving assembly 203 includes a second motor 2031, a second driving wheel 2032, a second driven wheel 2033, a second synchronous belt 2034, a second rotating shaft 2035, a cam 2036, a mounting bearing 2037, and a cam bracket 2038 with a second installation hole 2039. The cam bracket 2038 is arranged at the bottom of the magnetic platform 10, the second rotating shaft 2035 is arranged in the second installation hole 2039 through the mounting bearing 2037, the cam 2036 is fixedly arranged on the second rotating shaft 2035, the second driving wheel 2032 is arranged on an output shaft of the second motor 2031, the second synchronous belt 2034 is sleeved on the second driving wheel 2032 and the second driven wheel 2033, the second driven wheel 2033 is fixedly arranged on the second rotating shaft 2035, and a top of the cam 2036 abuts the lifting rod 202. As shown in FIG. 5, the second motor 2031 can be fixed on the magnetic platform 10 through a fixed block. In an embodiment, the ascending driving assembly 203 includes two cams 2036, two cam brackets 2038, two mounting bearings 2037, and two lifting rods 202. The two cams 2036, the two cam brackets 2038, and the two mounting bearings 2037 are symmetrically arranged on the second rotating shaft 2035. The two lifting rods 202 are arranged in parallel, and top ends of the two lifting rods 202 are connected to the lifting assembly 201. The two cams 2036 respectively abut bottom ends of the two lifting rods 202.

Furthermore, the turning on the ascending driving assembly 203 to drive the lifting rod 202 and thus to drive the lifting assembly 201 and the cover plate 301 to slide up along the lifting through-hole 1011 through the lifting rod 202 includes: turning on the second motor 2031 to drive the cam 2036 to sequentially rotate through the second driving wheel 2032, the second synchronous belt 2034, the second driven wheel 2033, and the second rotating shaft 2035, and driving the lifting assembly 201 and the cover plate 301 to slide up along the lifting through-hole 1011 through the lifting rod 202 by a rotation of the cam 2036. That is, after the second motor 2031 starts to rotate, the second motor 2031 drives the second driving wheel 2032 to rotate, and then drives the second driven wheel 2033 to rotate through the second synchronous belt 2034. The rotation of the second driven wheel 2033 drives the second rotating shaft 2035 and the two cams 2036 arranged on the second rotating shaft 2035 to rotate. At this time, the lifting rod 202 abutting the cam 2036 moves up with the rotation of the cam 2036, thereby driving the lifting assembly 201 and the cover plate 301 to ascend as a whole. At this time, if the second adsorption portion 303 is an electromagnet as described in the above embodiment, the electromagnet of the second adsorption portion 303 can be powered off, thus, the cover plate 301 ascends with the lifting assembly 201; when the iron block of the first adsorption portion 302 is adsorbed to the electromagnet of the second adsorption portion 303, it is confirmed that the cover plate 301 is closed.

Furthermore, as shown in FIG. 5, the ascending driving assembly 203 further includes an induction block 2042 arranged on the second rotating shaft 2035 and a photoelectric sensor 2043 arranged on the magnetic platform 10. The photoelectric sensor 2043 is used to determine that the cover plate 301 has ascended to a preset height when sensing that the second rotating shaft 2035 drives the induction block 2042 to rotate to a position facing the photoelectric sensor 2043. The preset height refers to the highest height to which the lifting assembly 201 and the cover plate 301 can be driven to ascend by the lifting rod 202. The preset height can be set according to the size of the liquid bag 50, and can be achieved by changing the size and shape of the cam 2036 according to the needs.

In one embodiment, as shown in FIG. 5, the ascending driving assembly 203 further includes a bearing follower 2040 and a fixing plate 2041. The bearing follower 2040 is arranged at a bottom end of the lifting rod 202 through the fixing plate 2041, and the cam 2036 abuts the bearing follower 2040. That is, the bearing follower 2040 can better transmit the lifting force generated by the rotation of the cam 2036 to the lifting rod 202, thereby allowing the lifting rod 202 to move upward more stably and more controllably.

In one embodiment, as shown in FIGS. 5 and 6, a guide through-hole 1012 is formed in the magnetic platform 10. The pressing mechanism 40 includes a guide shaft 401, a spring 402, and a linear bearing 403 arranged in the guide through-hole 1012. A stopper 4011 is arranged on a bottom end of the guide shaft 401, and a top end of the guide shaft 401 is connected to the lifting assembly 201 through the linear bearing 403. The spring 402 is sleeved on the guide shaft 401, and both ends of the spring 402 respectively abut the linear bearing 403 and the stopper 4011. Furthermore, the controlling the pressing mechanism 40 to drive the closed covering mechanism 30 to move downwards includes: turning off the ascending driving assembly 203, thereby driving the guide shaft 401 to move downwards along the guide through-hole 1012 through an elastic force of the spring 402, and driving the lifting assembly 201 and the cover plate 301 to move downwards. That is, in this embodiment, the ascending driving assembly 203 with the cam 2036 can only drive the lifting rod 202 to ascend, and does not have a downward pulling force to pull down the lifting assembly 201. Therefore, the pressing mechanism 40 is provided with the spring 402 being in a compressed state. After the second motor 2031 of the ascending driving assembly 203 is turned off (the second motor 2031 is reset), the spring 402 restores to its original state and applies a downward pulling force to the lifting assembly 201 through the guide shaft 401 (the spring 402 drives the guide shaft 401 to move down along the guide through-hole 1012), thereby driving the lifting assembly 201 and the cover plate 301 to move downwards. Understandably, if the guide shaft 401, the spring 402, and the linear bearing 403 are used as a pressing assembly, the pressing mechanism 40 can include multiple pressing assemblies arranged on the magnetic platform 10, such as four pressing assemblies arranged at the four corners of the square magnetic platform 10. In this way, when the four springs 402 of the four pressing assemblies simultaneously apply the downward pulling force to the lifting assembly 201, the cover plate 301 presses the liquid bag 50 towards the direction of the magnetic platform 10 as the lifting assembly 201 moves downwards, gradually flattening the liquid bag 50 and evenly distributing the liquid on the magnetic platform 10. In the present disclosure, the liquid bag 50 is flattened by the spring 402 instead of the second motor 2031. On the one hand, the liquid bag 50 can be evenly flattened and attached to the magnetic platform 10 by the spring 402. On the other hand, the elastic force of the spring 402 is uniform and gentle, and does crush the cells inside the liquid bag 50.

In one embodiment, as shown in FIG. 2, in step S20, the controlling the magnetic platform 10 to swing according to preset swing parameters includes steps as follows.

S201, obtaining the preset mixing parameters including a mixing angle and a mixing duration. The mixing angle is ± 35 degrees (which means that the magnetic platform 106 swings towards both opposite sides thereof, and the angle at which the magnetic platform 106 swings towards one side is within the range of 0-35 degrees; if the swing angle exceeds 35 degrees, the immunomagnetic cells may be killed, thus a maximum absolute value of the mixing angle is 35 degrees). Therefore, the maximum absolute value of the mixing angle is 35 degrees. In an embodiment, the mixing angle is ± 25 degrees. And the mixing duration can be 25 S-35 S; in an embodiment, the mixing duration is 30 S.

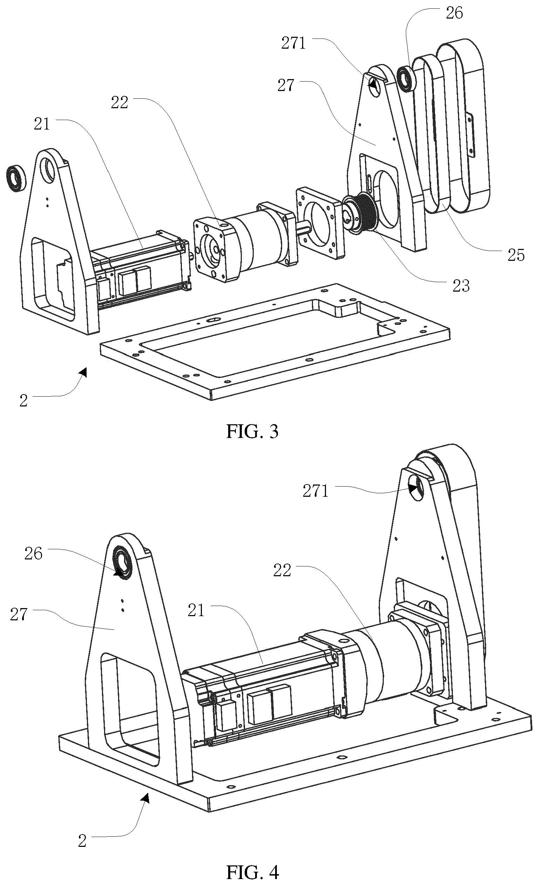

S202, controlling the mixing device 2 to continuously swing the magnetic platform 10 at the mixing angle for the mixing duration, such that the immunomagnetic cells are evenly adsorbed on the magnetic platform 10, and other substances in the liquid bag 50 except for the adsorbed immunomagnetic cells are suspended in the liquid as the magnetic platform 10 swings. Understandably, in an embodiment, as shown in FIGS. 3, 4, and 5, the mixing device 2 includes a first motor 21, a speed reducer 22, a first driving wheel 23, a first driven wheel 24, a first synchronous belt 25, and a synchronous bracket 27. The speed reducer 22 is connected to an output shaft of the first motor 21 to increase an output torque while reducing a speed of the first motor 21. The first driving wheel 23 is connected to a rotation shaft 2014 of the speed reducer 22. The first synchronous belt 25 is sleeved on the first driving wheel 23 and the first driven wheel 24, and the first driven wheel 24 is arranged on the magnetic platform 10. The magnetic platform 10 is rotatably arranged on the synchronous bracket 27. The first driving wheel 23 is used to drive the first driven wheel 24 to rotate through the first synchronous belt 25 under the driving of the speed reducer 22, thereby causing the magnetic platform 10 to swing around a fulcrum of the synchronous bracket 27 to mix the liquid in the liquid bag 50. In an embodiment, the first motor 21 can be a stepper motor. Due to the small torque output by the first motor 21, the first motor 21 cannot drive the magnetic platform 10 to swing. Therefore, the present disclosure reduces the speed of the first motor 21 through the speed reducer 22, thereby increasing the output torque of the first motor 21 and thus driving the magnetic platform 10 to swing at a certain angle through the linkage of the first driving wheel 23, the first synchronous belt 25, and the first driven wheel 24. Through the swinging of the magnetic platform 10, the liquid in the liquid bag 50 loaded on the magnetic platform 10 is mixed, thereby achieving uniform adsorption of the immunomagnetic cells on the magnetic platform 10, and allowing other substances in the liquid bag 50 except for the adsorbed immunomagnetic cells to be suspended in the liquid as the magnetic platform 10 swings.

In one embodiment, as shown in FIGS. 3 to 5, the mixing device 2 further includes a bearing 26, a first installation hole 271 is formed in the synchronous bracket 27, and the magnetic platform 10 includes a first rotating shaft 103 rotatably arranged in the first installation hole 271 through the bearing 26. The first driven wheel 24 is arranged on the first rotating shaft 103. That is, when the first motor 21 drives the magnetic platform 10 to swing through the linkage of the speed reducer 222, the first driving wheel 23, the first synchronous belt 25, and the first driven wheel 24, a fulcrum of the swinging of the magnetic platform 10 is the bearing 26 arranged on the synchronous bracket 27, that is, an outer ring of the bearing 26 is fixed in the first installation hole 271, and an inner ring of the bearing 26 is fixed on the first rotating shaft 103. With the swinging of the magnetic platform 10, the first rotating shaft 103 rotates to drive the inner ring of the bearing 26 to rotate relative to the outer ring thereof. Understandably, by controlling a rotation direction of the first motor 21 to be different, the magnetic platform 10 can be controlled to swing in an opposite direction with the bearing 26 as the fulcrum. By controlling the speed of the first motor 21, the swing angle of the magnetic platform 10 can be controlled. Furthermore, after the liquid is evenly mixed due to the swinging of the magnetic platform 10, the magnetic platform 10 is controlled to stay at a preset tilt angle, such that the liquid in the liquid bag 50 can flow out from the liquid outlet after the magnetic bead sorting is completed.

Furthermore, in step S202, the controlling the mixing device 2 to continuously swing the magnetic platform 10 at the mixing angle includes steps as follows.

Determining operating parameters of the first motor 21 based on the mixing angle. That is, if the required mixing angle for the magnetic platform 10 is determined, the real-time operating parameters of the first motor 21 can be determined based on historical experimental data (which includes the correlation between historical operating parameters of the first motor 21 and historical mixing angles) and the mixing angle. Firstly, the historical mixing angle matching the mixing angle can be determined in the historical experimental data, and then the historical operating parameters of the first motor 21 associated with the determined historical mixing angle can be determined as the real-time operating parameters of the first motor 21.

Turning on the first motor 21 to drive the speed reducer 22 with the operating parameters to drive the first driving wheel 23 to rotate. The first driving wheel 23 drives the first driven wheel 24 to rotate through the first synchronous belt 25, thereby driving the magnetic platform 10 to swing around the connection point with the synchronous bracket 27. In the present disclosure, the first motor 21 firstly drives the speed reducer 22 to rotate, which in turn drives the first driving wheel 23, the first synchronous belt 25, and the first driven wheel 24 to rotate together, causing the magnetic platform 10 to swing around the first rotating shaft 103 with the bearing 26 as the fulcrum, and the swing angular velocity, the swing angle, and the angular acceleration are respectively matched with each parameter in the swing parameters mentioned above. Furthermore, as shown in FIG. 5, the mixing device 2 also includes a dual axis inclination sensor 28 arranged on the magnetic platform 10, which is used to detect the swing parameters of the magnetic platform 10. The dual axis inclination sensor 28 can detect the swing angle, the swing angular velocity, the angular acceleration, and the staying angle after the swinging during the entire swinging process of the mixing device 2.

Furthermore, in step S202, after controlling the mixing device 2 to continuously swing the magnetic platform 10 at the mixing angle for the mixing duration, and before performing the waste liquid discharge operation in step S30, the magnetic bead sorting method further includes steps follows.

Placing the magnetic platform 10 horizontally for a preset duration. The preset duration can be set to 5-20 minutes according to needs; in an embodiment, the preset duration is 10 minutes.

Obtaining first gentle swing parameters which include a first gentle swing angle and a first gentle swing duration. An absolute value of the first gentle swing angle is smaller than an absolute value of the mixing angle. For example, when the mixing angle is ± 25 degrees, the first gentle swing angle is plus or minus 20 degrees.

Controlling the mixing device 2 to continuously swing the magnetic platform 10 at the first gentle swing angle for the first gentle swing duration, such that other substances in the liquid bag 50, except for the adsorbed immunomagnetic cells, are suspended in the liquid. In the present disclosure, when the magnetic platform 10 swings at the mixing angle, the magnetic platform 10 can flatten the accumulated immunomagnetic cells to be more uniform, while allowing other substances in the liquid bag 50 except for the adsorbed immunomagnetic cells to be suspended in the liquid. In this embodiment, when the magnetic platform 10 swings at the first gentle swing angle, the swinging force is relatively gentle and does not affect the immunomagnetic cells which have been already adsorbed by the magnetic platform 10. Instead, as the magnetic platform 10 swings, other substances adhered to the adsorbed immunomagnetic cells are shaken to detach from the immunomagnetic cells, such that the other substances can be suspended in the liquid, facilitating the discharge of the other substances during the waste liquid discharge operation in step S30.

Furthermore, in step S30, the performing the waste liquid discharge operation includes steps as follows.

Controlling the magnetic platform 10 to stay at a first preset staying angle, such that the liquid bag 50 tilts downwards from a liquid inlet thereof towards a liquid outlet thereof. In this embodiment, after the magnetic platform 10 is controlled to swing and mix the liquid in the liquid bag 50 according to the preset swing parameters, the first preset staying angle allows the liquid outlet of the liquid bag 50 on the stationary magnetic platform 10 to be lower than the liquid inlet of the liquid bag 50, thereby allowing the liquid in the liquid bag 50 to flow out from the liquid outlet thereof. In an embodiment, after the magnetic platform 10 stops swinging, the first preset staying angle is the angle between the magnetic platform 10 and a horizontal plane, which can be 10-30 degrees. In an embodiment, the first preset staying angle is 20 degrees.

Performing a first switch operation to open the waste liquid discharge pipeline. The waste liquid discharge pipeline includes a pump and a bubble sensor connected between the waste liquid bag 50 and the liquid outlet of the liquid bag 50. That is, the first switch operation refers to opening all the closed valves along the line including the waste liquid bag 50, the pump, the bubble sensor and the liquid bag 50 in the waste liquid discharge pipeline, and simultaneously turning on the pump (in an embodiment, a peristaltic pump) and the bubble sensor. At this time, it is considered that the waste liquid discharge pipeline is opened.

Discharging the liquid in the liquid bag 50, except for the adsorbed immunomagnetic cells, into the waste liquid bag 50 through the waste liquid discharge pipeline. In an embodiment, the pump in the waste liquid discharge pipeline extracts the liquid in the liquid bag 50, except for the adsorbed immunomagnetic cells, into the waste liquid bag 50. The bubble sensor detects the bubbles in the liquid in the waste liquid discharge pipeline and determines whether there is still liquid flowing through the waste liquid discharge pipeline based on the detection results.

Confirming the completion of the waste liquid discharge when the bubble sensor determines that no liquid is passing through the waste liquid discharge pipeline. That is, when it is determined that no liquid is passing through the waste liquid discharge pipeline, it indicates that the liquid in the liquid bag 50 has been drained, and therefore the waste liquid discharge operation can be considered complete.

Furthermore, in step S40, the performing a cleaning operation on remaining immunomagnetic cells in the liquid bag 50 includes:
Controlling the magnetic platform 10 to stay at a second preset staying angle, such that the liquid bag 50 tilts upwards from the liquid inlet thereof towards the liquid outlet thereof. A direction of the second preset staying angle is opposite to that of the first preset staying angle. In this embodiment, the second preset staying angle allows the liquid outlet of the liquid bag 50 on the stationary magnetic platform 10 to be higher than the liquid inlet of the liquid bag 50, such that the cleaning solution at a subsequent cleaning solution inlet can flow into the liquid bag 50 from the liquid inlet and stays near the liquid inlet, thereby facilitating the penetration of the cleaning solution into various corners of the liquid bag 50 from the liquid inlet. In an embodiment, when the magnetic platform 10 stays at the second preset staying angle, the angle between the magnetic platform 10 and the horizontal plane is the second preset staying angle, which can be 10-30 degrees and be 15-20 degrees in an embodiment.

Performing a second switch operation to open the cleaning pipeline. The second switch operation includes turning off the pump and a channel between the bubble sensor and the waste liquid bag 50, and connecting the pump and the bubble sensor between a cleaning solution liquid inlet and the liquid inlet of the liquid bag 50. That is, the second switch operation refers to closing the valve between the pump and the bubble sensor in the waste liquid discharge pipeline and the waste liquid bag 50, such that neither the pump nor the bubble sensor is connected to the waste liquid bag 50. At the same time, the pump and the bubble sensor are also turned off, that is, the waste liquid discharge pipeline is closed. At this point, the closed valves along the line including the cleaning solution liquid inlet, the pump, the bubble sensor, and the liquid bag 50 are opened, and then the pump (in an embodiment, a peristaltic pump) and the bubble sensor are turned on. At this point, it is considered that the cleaning pipeline is opened.

Inputting a first preset volume of the cleaning solution into the liquid bag 50 through the cleaning pipeline. The first preset volume can be 50-300 ml and can be 100 ml in an embodiment.

Obtaining second gentle swing parameters including a second gentle swing angle and a second gentle swing duration. An absolute value of the second gentle swing angle is smaller than an absolute value of the mixing angle. For example, when the mixing angle is ± 25 degrees, the second gentle swing angle is plus or minus 20 degrees.

Controlling the mixing device 2 to continuously swing the magnetic platform 10 at the second gentle swing angle for the second gentle swing duration, and then performing the waste liquid discharge operation again. In the present disclosure, when the magnetic platform 10 swings at the mixing angle, the magnetic platform 10 can allow the accumulated immunomagnetic cells to spread out more evenly. However, in this embodiment, when the magnetic platform 10 swings at the second gentle swing angle, the swinging force is relatively gentle, and the magnetic platform 10 does not shake the immunomagnetic cells which have been already adsorbed by the magnetic platform 10. Instead, the magnetic platform 19 shakes other substances adhered to the already adsorbed immunomagnetic cells until the other substances detach from the adsorbed immunomagnetic cells, such the other substances are suspended in the cleaning solution for easy discharge during the waste liquid discharge operation. Through the above cleaning operation, the purity of the immunomagnetic cells adsorbed in the final liquid bag 50 can be further ensured. Understandably, the steps for performing the waste liquid discharge operation again can refer to the description of the waste liquid discharge operation in the above embodiments, which will not be repeated here. The difference between the two waste liquid discharge operations lies in that, when the waste liquid discharge operation is performed again, the valve between the pump and the bubble sensor and the cleaning solution liquid inlet in the cleaning pipeline needs to be closed at first in the first switch operation, such that neither the pump nor the bubble sensor is connected to the cleaning solution liquid inlet; the pump and the bubble sensor are also turned off, that is, the cleaning pipeline is closed. And then, all the closed valves on the line including the waste liquid bag 50, the pump, the bubble sensor, and the liquid bag 50 are opened, and the pump (in an embodiment, a peristaltic pump) and the bubble sensor are turned on. At this time, it is considered that the waste liquid discharge pipeline is opened again.

Furthermore, in step S50, the injecting resuspension into the liquid bag 50 and removing the liquid bag 50 containing immunomagnetic cells includes steps as follows.

Adjusting the magnetic platform 10 to be parallel to the horizontal plane. That is, after performing the waste liquid discharge operation again, the magnetic platform 10 is firstly reset to a horizontal position for subsequent operations.

Performing a third switch operation to open a resuspension input pipeline. The third switch operation includes turning off the pump and the channel between the bubble sensor and the waste liquid bag 50, and connecting the pump and the bubble sensor between a resuspension inlet and the liquid inlet of the liquid bag 50. That is, the third switch operation refers to closing the valve between the pump and the bubble sensor in the waste liquid discharge pipeline and the waste liquid bag 50, such that neither the pump nor the bubble sensor is connected to the waste liquid bag 50; the pump and the bubble sensor are also turned off, that is, the waste liquid discharge pipeline is closed. And then, all the closed valves on the line including the resuspension inlet, the pump, the bubble sensor, and the liquid bag 50 are opened, and the pump (in an embodiment, a peristaltic pump) and the bubble sensor are turned on. At this time, it is considered that the resuspension input pipeline is opened.

Inputting a second preset volume of the resuspension into the liquid bag 50 through the resuspension input pipeline. Understandably, the second preset volume can be set according to demand, such as 0-300 ml.

Performing a fourth switch operation including turning off the pump and the channel between the bubble sensor and the resuspension inlet, and controlling the pump and the bubble sensor to communicate with the atmosphere through a sterile air filter. That is, the fourth switch operation refers to closing the valve between the pump and the bubble sensor in the resuspension input pipeline and the resuspension inlet, such that neither the pump nor the bubble sensor is connected to the resuspension inlet. At this time, all the closed valves on the line including the sterile air filter, the pump, the bubble sensor, and the liquid bag 50 are opened, and it is considered that the resuspension input pipeline is opened. Furthermore, the liquid bag 50 can communicate with the atmosphere through the pump, the bubble sensor, and the sterile air filter. Under the atmospheric pressure, the resuspension remaining in the resuspension input pipeline can enter the liquid bag 50 by the pump and the resuspension input pipeline is empty, without wasting the resuspension in the resuspension input pipeline. The price of the resuspension is high, and the above operation in this embodiment can greatly reduce the cost of the magnetic bead sorting process.

When the bubble sensor determines that that no liquid is passing through the channel between the bubble sensor and the resuspension inlet, it is determined that the resuspension is completely injected into the liquid bag. Understandably, when the bubble sensor detects bubbles in the pipeline and determines that there is no more liquid flowing through the resuspension input pipeline based on the detection results, it can be considered that the resuspension has been completely injected into the liquid bag 50.

Performing thermal sealing on the liquid outlet and the liquid inlet of the liquid bag 50, and removing the liquid bag 50 containing the immunomagnetic cells from the magnetic platform 10. In this embodiment, after the resuspension input pipeline is empty, both the liquid outlet and the liquid inlet of the liquid bag 50 are thermally sealed, that is, a thermal sealer device is used to seal the liquid outlet and the liquid inlet thermally. Then, the liquid bag 50 is removed from the magnetic platform 10 to complete the magnetic bead sorting process. The immunomagnetic cells in the resuspension in the liquid bag 50 are the target objects for collection. At this point, the collection is completed and subsequent culturing can be carried out.

Furthermore, the inputting the second preset volume of resuspension into the liquid bag 50 through the resuspension input pipeline includes steps as follows.

Injecting the resuspension into the liquid bag 50 through the resuspension input pipeline. That is, in this embodiment, after the resuspension input pipeline is opened, the resuspension can be injected into the liquid bag 50 through the opened resuspension input pipeline.

Obtaining weight change information of the liquid bag 50 through a weighing sensor, and determining an actual volume of the injected resuspension based on the weight change information of the liquid bag 50; or, obtaining volume to rotation data corresponding to the pump, and determining the actual volume of the injected resuspension based on an actual rotation number of the pump and the volume to rotation number data. That is, in this embodiment, the difference between the current weight and the original weight of the liquid bag 50 can be determined by the weight change information of the liquid bag 50, and then the actual volume of the injected resuspension can be determined based on this difference and the density of the resuspension. In another embodiment, the actual volume of the injected resuspension that is, the volume of the resuspension input into the liquid bag 50 corresponding to each rotation of the pump obtained in historical experiments, that is, the volume to rotation number data, can be determined based on historical data. After obtaining the actual rotation number of the pump, the actual volume of the injected resuspension can be determined.

When the actual volume reaches the second preset volume, confirming that the resuspension of the second preset volume has been input into the liquid bag 50. That is, since only the second preset volume of the resuspension needs to be injected, after determining that the second preset volume of the resuspension has been injected according to the above method, it can be confirmed that the second preset volume of the resuspension has been input into the liquid bag 50, and then the subsequent fourth switch operation and other steps can be performed. It can be understood, although in the fourth switch operation mentioned above, the remaining resuspension in the resuspension input pipeline can enter the liquid bag 50, this part of the resuspension is not included in the actual volume before the fourth switch operation is performed. Therefore, the second preset volume can be set slightly smaller than the actual volume of the resuspension that needs to be injected into the liquid bag 50 (the difference between the two volumes can be a constant value obtained from the experiment). In this way, the required volume of the resuspension can be injected to the liquid bag 50 by adding the second preset volume and the remaining resuspension entering the liquid bag 50 from the resuspension input pipeline.

It should be understood that the size of the sequence numbers of each step in the above embodiments does not imply the order of execution. The order of execution of each process should be determined by its function and internal logic, and should not constitute any limitation on the implementation process of the present disclosure.

The present disclosure also provides a magnetic bead sorting device, including the pressing device 1, the mixing device 2, and a controller for performing the magnetic bead sorting method described above, wherein the controller is connected to the pressing device 1 and the mixing device 2.

In an embodiment, the controller is used to perform the following steps:
placing an empty liquid bag on a magnetic platform, inputting a liquid containing immunomagnetic cells into the liquid bag, and adsorbing the immunomagnetic cells in the liquid bag through the magnetic platform;
controlling the magnetic platform to swing according to preset swing parameters to evenly distribute the immunomagnetic cells adsorbed by the magnetic platform, while allowing other substances in the liquid bag except for the adsorbed immunomagnetic cells to be suspended in the liquid;
performing a waste liquid discharge operation to allow the liquid in the liquid bag, except for the adsorbed immunomagnetic cells, to flow into a waste liquid bag;
performing a cleaning operation on remaining immunomagnetic cells in the liquid bag;
after injecting a resuspension into the liquid bag and removing the liquid bag containing the immunomagnetic cells, confirming that a magnetic bead sorting process is complete.

In an embodiment, the placing an empty liquid bag on a magnetic platform, inputting a liquid containing immunomagnetic cells into the liquid bag, and adsorbing the immunomagnetic cells in the liquid bag through the magnetic platform includes:
placing the empty liquid bag on the magnetic platform of a pressing device, wherein the pressing device includes a covering mechanism arranged on the magnetic platform and a pressing mechanism connected to the covering mechanism;
inputting the liquid containing the immunomagnetic cells into the liquid bag;
after the covering mechanism is closed, controlling the pressing mechanism to drive the closed covering mechanism to move downwards to flatten the liquid bag placed in an accommodating space between the magnetic platform and the covering mechanism, thereby increasing a contact surface between a bottom surface of the liquid bag and the magnetic platform, and then adsorbing the immunomagnetic cells in the liquid bag onto the contact surface through the magnetic platform.

In an embodiment, the pressing device further includes a lifting mechanism connected to the covering mechanism;
after the inputting the liquid containing the immunomagnetic cells into the liquid bag, the magnetic bead storing method further includes:
when the covering mechanism is pushed up by the liquid bag which is expanded and cannot be closed, controlling the lifting mechanism to drive the covering mechanism to ascend, thereby increasing the accommodating space between the magnetic platform and the covering mechanism for placing the liquid bag;
after the covering mechanism ascends to a preset height at which the covering mechanism can be closed, controlling the covering mechanism to close;
controlling the pressing mechanism to drive the closed covering mechanism to move downwards, thereby flattening the liquid bag placed in the accommodating space, increasing the contact surface between the bottom surface of the liquid bag and the magnetic platform, and then adsorbing the immunomagnetic cells in the liquid bag onto the contact surface through the magnetic platform.

In an embodiment, the controlling the magnetic platform to swing according to preset swing parameters includes:
obtaining preset mixing parameters including a mixing angle and a mixing duration;
controlling a mixing device to continuously swing the magnetic platform at the mixing angle for the mixing duration, such that the immunomagnetic cells are evenly adsorbed on the magnetic platform and other substances in the liquid bag, except for the adsorbed immunomagnetic cells, are suspended in the liquid as the magnetic platform swings.

In an embodiment, after the mixing device controls the magnetic platform to continuously swing at the mixing angle for the mixing duration, and before the performing a waste liquid discharge operation, the controller is further used to perform the following steps:
placing the magnetic platform horizontally for a preset duration;
obtaining first gentle swing parameters including a first gentle swing angle and a first gentle swing duration, wherein an absolute value of the first gentle swing angle is smaller than an absolute value of the mixing angle;
controlling the mixing device to continuously swing the magnetic platform at the first gentle swing angle for the first gentle swing duration, such that other substances in the liquid bag, except for the adsorbed immunomagnetic cells, are suspended in the liquid.

In an embodiment, the mixing device includes a first motor, a speed reducer, a first driving wheel, a first driven wheel, a first synchronous belt, and a synchronous bracket; the speed reducer is connected to an output shaft of the first motor to increase an output torque of the first motor while reducing a speed of the first motor; the first driving wheel is connected to a rotating shaft of the speed reducer, and the first synchronous belt is sleeved on the first driving wheel and the first driven wheel; the first driven wheel is arranged on the magnetic platform; the magnetic platform is rotatably arranged on the synchronous bracket;
the controlling a mixing device to continuously swing the magnetic platform at the mixing angle includes:
determining operating parameters of the first motor based on the mixing angle;
Turning on the first motor to drive the speed reducer with the operating parameters to drive the first driving wheel to rotate; driving the first driven wheel to rotate by the first driving wheel through the first synchronous belt, thereby driving the magnetic platform to swing around a connection point with the synchronous bracket.

In an embodiment the performing a waste liquid discharge operation includes:
controlling the magnetic platform to stay at a first preset staying angle, such that the liquid bag tilts downwards from a liquid inlet of the liquid bag towards a liquid outlet of the liquid bag;
performing a first switch operation to open a waste liquid discharge pipeline, wherein the waste liquid discharge pipeline includes a pump and a bubble sensor connected between the waste liquid bag and the liquid outlet of the liquid bag;
discharging the liquid in the liquid bag, except for the adsorbed immunomagnetic cells, into the waste liquid bag through the waste liquid discharge pipeline; and
confirming the waste liquid discharge operation is complete when the bubble sensor determines that no liquid is passing through the waste liquid discharge pipeline.

In an embodiment, the performing a cleaning operation on remaining immunomagnetic cells in the liquid bag includes:
controlling the magnetic platform to stay at a second preset staying angle, such that the liquid bag tilts upwards from the liquid inlet of the liquid bag towards the liquid outlet of the liquid bag, wherein a direction of the second preset staying angle is opposite to that of the first preset staying angle;
performing a second switch operation to open a cleaning pipeline, wherein the second switch operation includes turning off the pump and a channel between the bubble sensor and the waste liquid bag, and connecting the pump and the bubble sensor between a cleaning solution liquid inlet and the liquid inlet of the liquid bag;
inputting a first preset volume of cleaning solution into the liquid bag through the cleaning pipeline;
obtaining second gentle swing parameters including a second gentle swing angle and a second gentle swing duration;
controlling the mixing device to continuously swing the magnetic platform at the second gentle swing angle for the second gentle swing duration, and then performing the waste liquid discharge operation again.

In an embodiment, the injecting a resuspension into the liquid bag and removing the liquid bag containing immunomagnetic cells includes:
adjusting the magnetic platform to be parallel to a horizontal plane;
performing a third switch operation to open a resuspension input pipeline, wherein the third switch operation includes turning off the pump and the channel between the bubble sensor and the waste liquid bag, and connecting the pump and the bubble sensor between a resuspension inlet and the liquid inlet of the liquid bag;
inputting a second preset volume of the resuspension into the liquid bag through a resuspension input pipeline;
performing a fourth switch operation including turning off the pump and a channel between the bubble sensor and the resuspension inlet, and controlling the pump and the bubble sensor to communicate with atmosphere through a sterile air filter;
when the bubble sensor determines that no liquid is passing through the channel between the bubble sensor and the resuspension inlet, it is determined that the resuspension is completely injected into the liquid bag;
after performing thermal sealing on the liquid outlet and liquid inlet of the liquid bag, removing the liquid bag containing the immunomagnetic cells from the magnetic platform.

In an embodiment, the inputting a second preset volume of the resuspension into the liquid bag through a resuspension input pipeline includes:
injecting the resuspension into the liquid bag through the resuspension input pipeline;
obtaining weight change information of the liquid bag through a weighing sensor, and determining an actual volume of the injected resuspension based on the weight change information of the liquid bag, or, obtaining volume to rotation number data corresponding to the pump, and determining the actual volume of the injected resuspension based on an actual rotation number of the pump and the volume to rotation number data;
when the actual volume reaches the second preset volume, confirming that the resuspension of the second preset volume has been input into the liquid bag.

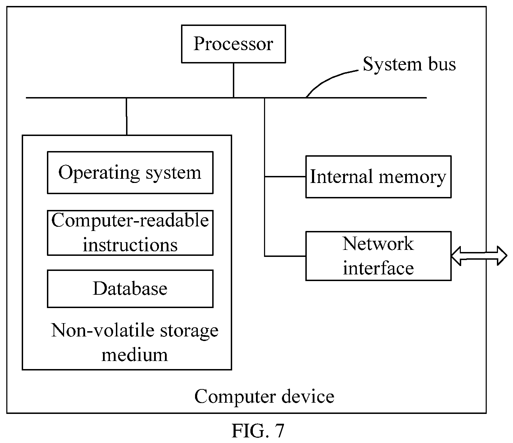

For more specific limitations on magnetic bead sorting equipment and controllers, please refer to the limitations on the magnetic bead sorting method mentioned above, which is not repeated here. The various modules in the above controller can be fully or partially implemented through software, hardware, and their combinations. The above modules can be embedded in hardware form or independent of the processor in the computer device, or stored in software form in the memory of the computer device, so that the processor can call and execute the corresponding operations of the above modules. Understandably, the controller can be regarded as one or more computer devices, as shown in FIG. 7, which include a processor, a memory, a network interface, and a database connected through a system bus. The processor of the computer device is used to provide computing and control capabilities. The memory of the computer device includes a computer-readable storage medium and an internal memory. The computer-readable storage medium stores an operating system, computer-readable instructions, and a database. The internal memory provides an environment for the execution of operating systems and computer-readable instructions in computer-readable storage medium. The database of the computer device is used to store the data used for the magnetic bead sorting method in the above embodiments. The network interface of the computer device is used to communicate with external terminals through a network connection. This computer-readable instruction is executed by a processor to implement the magnetic bead sorting method. The computer-readable storage medium provided in this embodiment include a non-volatile computer-readable storage medium and a volatile computer-readable storage medium.

In one embodiment, one or more computer-readable storage media storing computer-readable instructions are provided, and the computer-readable storage media provided in this embodiment include non-volatile computer-readable storage media and volatile computer-readable storage media; The computer-readable storage medium stores computer-readable instructions which, when executed by one or more processors, enable one or more processors to implement the magnetic bead sorting method described above.

When being executed by one or more processors, the computer-readable instructions cause the one or more processors to implement the following steps:
placing an empty liquid bag on a magnetic platform, inputting a liquid containing immunomagnetic cells into the liquid bag, and adsorbing the immunomagnetic cells in the liquid bag through the magnetic platform;
controlling the magnetic platform to swing according to preset swing parameters to evenly distribute the immunomagnetic cells adsorbed by the magnetic platform, while allowing other substances in the liquid bag except for the adsorbed immunomagnetic cells to be suspended in the liquid;
performing a waste liquid discharge operation to allow the liquid in the liquid bag, except for the adsorbed immunomagnetic cells, to flow into a waste liquid bag;
performing a cleaning operation on remaining immunomagnetic cells in the liquid bag;
after injecting a resuspension into the liquid bag and removing the liquid bag containing the immunomagnetic cells, confirming that a magnetic bead sorting process is complete.

In an embodiment, the placing an empty liquid bag on a magnetic platform, inputting a liquid containing immunomagnetic cells into the liquid bag, and adsorbing the immunomagnetic cells in the liquid bag through the magnetic platform includes:
placing the empty liquid bag on the magnetic platform of a pressing device, wherein the pressing device includes a covering mechanism arranged on the magnetic platform and a pressing mechanism connected to the covering mechanism;
inputting the liquid containing the immunomagnetic cells into the liquid bag;
after the covering mechanism is closed, controlling the pressing mechanism to drive the closed covering mechanism to move downwards to flatten the liquid bag placed in an accommodating space between the magnetic platform and the covering mechanism, thereby increasing a contact surface between a bottom surface of the liquid bag and the magnetic platform, and then adsorbing the immunomagnetic cells in the liquid bag onto the contact surface through the magnetic platform.

In an embodiment, the pressing device further includes a lifting mechanism connected to the covering mechanism;
after the inputting the liquid containing the immunomagnetic cells into the liquid bag, the magnetic bead storing method further includes:
when the covering mechanism is pushed up by the liquid bag which is expanded and cannot be closed, controlling the lifting mechanism to drive the covering mechanism to ascend, thereby increasing the accommodating space between the magnetic platform and the covering mechanism for placing the liquid bag;
after the covering mechanism ascends to a preset height at which the covering mechanism can be closed, controlling the covering mechanism to close;
controlling the pressing mechanism to drive the closed covering mechanism to move downwards, thereby flattening the liquid bag placed in the accommodating space, increasing the contact surface between the bottom surface of the liquid bag and the magnetic platform, and then adsorbing the immunomagnetic cells in the liquid bag onto the contact surface through the magnetic platform.

In an embodiment, the controlling the magnetic platform to swing according to preset swing parameters includes:
obtaining preset mixing parameters including a mixing angle and a mixing duration;
controlling a mixing device to continuously swing the magnetic platform at the mixing angle for the mixing duration, such that the immunomagnetic cells are evenly adsorbed on the magnetic platform and other substances in the liquid bag, except for the adsorbed immunomagnetic cells, are suspended in the liquid as the magnetic platform swings.

In an embodiment, after the mixing device controls the magnetic platform to continuously swing at the mixing angle for the mixing duration, and before the performing a waste liquid discharge operation, the controller is further used to perform the following steps:
placing the magnetic platform horizontally for a preset duration;
obtaining first gentle swing parameters including a first gentle swing angle and a first gentle swing duration, wherein an absolute value of the first gentle swing angle is smaller than an absolute value of the mixing angle;
controlling the mixing device to continuously swing the magnetic platform at the first gentle swing angle for the first gentle swing duration, such that other substances in the liquid bag, except for the adsorbed immunomagnetic cells, are suspended in the liquid.

In an embodiment, the mixing device includes a first motor, a speed reducer, a first driving wheel, a first driven wheel, a first synchronous belt, and a synchronous bracket; the speed reducer is connected to an output shaft of the first motor to increase an output torque of the first motor while reducing a speed of the first motor; the first driving wheel is connected to a rotating shaft of the speed reducer, and the first synchronous belt is sleeved on the first driving wheel and the first driven wheel; the first driven wheel is arranged on the magnetic platform; the magnetic platform is rotatably arranged on the synchronous bracket;
the controlling a mixing device to continuously swing the magnetic platform at the mixing angle includes:
determining operating parameters of the first motor based on the mixing angle;
Turning on the first motor to drive the speed reducer with the operating parameters to drive the first driving wheel to rotate; driving the first driven wheel to rotate by the first driving wheel through the first synchronous belt, thereby driving the magnetic platform to swing around a connection point with the synchronous bracket.

In an embodiment the performing a waste liquid discharge operation includes:
controlling the magnetic platform to stay at a first preset staying angle, such that the liquid bag tilts downwards from a liquid inlet of the liquid bag towards a liquid outlet of the liquid bag;
performing a first switch operation to open a waste liquid discharge pipeline, wherein the waste liquid discharge pipeline includes a pump and a bubble sensor connected between the waste liquid bag and the liquid outlet of the liquid bag;
discharging the liquid in the liquid bag, except for the adsorbed immunomagnetic cells, into the waste liquid bag through the waste liquid discharge pipeline; and
confirming the waste liquid discharge operation is complete when the bubble sensor determines that no liquid is passing through the waste liquid discharge pipeline.

In an embodiment, the performing a cleaning operation on remaining immunomagnetic cells in the liquid bag includes:
controlling the magnetic platform to stay at a second preset staying angle, such that the liquid bag tilts upwards from the liquid inlet of the liquid bag towards the liquid outlet of the liquid bag, wherein a direction of the second preset staying angle is opposite to that of the first preset staying angle;
performing a second switch operation to open a cleaning pipeline, wherein the second switch operation includes turning off the pump and a channel between the bubble sensor and the waste liquid bag, and connecting the pump and the bubble sensor between a cleaning solution liquid inlet and the liquid inlet of the liquid bag;
inputting a first preset volume of cleaning solution into the liquid bag through the cleaning pipeline;
obtaining second gentle swing parameters including a second gentle swing angle and a second gentle swing duration;
controlling the mixing device to continuously swing the magnetic platform at the second gentle swing angle for the second gentle swing duration, and then performing the waste liquid discharge operation again.

In an embodiment, the injecting a resuspension into the liquid bag and removing the liquid bag containing immunomagnetic cells includes:
adjusting the magnetic platform to be parallel to a horizontal plane;
performing a third switch operation to open a resuspension input pipeline, wherein the third switch operation includes turning off the pump and the channel between the bubble sensor and the waste liquid bag, and connecting the pump and the bubble sensor between a resuspension inlet and the liquid inlet of the liquid bag;
inputting a second preset volume of the resuspension into the liquid bag through a resuspension input pipeline;
performing a fourth switch operation including turning off the pump and a channel between the bubble sensor and the resuspension inlet, and controlling the pump and the bubble sensor to communicate with atmosphere through a sterile air filter;
when the bubble sensor determines that no liquid is passing through the channel between the bubble sensor and the resuspension inlet, it is determined that the resuspension is completely injected into the liquid bag;
after performing thermal sealing on the liquid outlet and liquid inlet of the liquid bag, removing the liquid bag containing the immunomagnetic cells from the magnetic platform.

In an embodiment, the inputting a second preset volume of the resuspension into the liquid bag through a resuspension input pipeline includes:
injecting the resuspension into the liquid bag through the resuspension input pipeline;
obtaining weight change information of the liquid bag through a weighing sensor, and determining an actual volume of the injected resuspension based on the weight change information of the liquid bag, or, obtaining volume to rotation number data corresponding to the pump, and determining the actual volume of the injected resuspension based on an actual rotation number of the pump and the volume to rotation number data;
when the actual volume reaches the second preset volume, confirming that the resuspension of the second preset volume has been input into the liquid bag.

For specific limitations on readable storage media, please refer to the limitations on magnetic bead sorting methods mentioned earlier, which will not be repeated here.

Those skilled in the art may understand that all or some of the processes of the method in the above embodiments may be implemented by instructing related hardware by using computer-readable instructions. The computer-readable instructions may be stored in a non-volatile computer-readable storage medium or a volatile computer-readable storage medium. When the computer-readable instructions are executed, the processes in the above method embodiments may be implemented. Any reference to the memory, storage, database, or other media used in the embodiments provided in the present disclosure may include a non-volatile and/or volatile memory. The non-volatile memory may include a read-only memory (ROM), a programmable ROM (PROM), an electrically programmable ROM (EPROM), an electrically erasable programmable ROM (EEPROM), or a flash memory. The volatile memory may include a random access memory (RAM) or an external cache memory. By way of illustration instead of limitation, the RAM is available in a plurality of forms, such as a static RAM (SRAM), a dynamic RAM (DRAM), a synchronous DRAM (SDRAM), a double data rate SDRAM (DDRSDRAM), an enhanced SDRAM (ESDRAM), a synchlink (Synchlink) DRAM (SLDRAM), a Rambus (Rambus) direct RAM (RDRAM), a direct Rambus dynamic RAM (DRDRAM), and a Rambus dynamic RAM (RDRAM).

Those skilled in the art may clearly understand that, for convenience and brevity of description, only the division of the above functional units or modules is used as an example for description. In actual applications, the above functions may be allocated to different functional units or modules for implementation as required, that is, an internal structure of the device is divided into different functional units or modules to implement all or some of the functions described above.

The above embodiments are only used to illustrate the technical solution of the present disclosure, and not to limit it; Although the present disclosure has been described in detail with reference to the aforementioned embodiments, those skilled in the art should understand that they can still modify the technical solutions described in the aforementioned embodiments, or equivalently replace some of the technical features; And these modifications or substitutions do not deviate from the essence and scope of the technical solutions of the embodiments of the present disclosure, and should be included in the scope of protection of the present disclosure.

## Claims

1. A magnetic bead sorting method, comprising:
placing an empty liquid bag on a magnetic platform, inputting a liquid containing immunomagnetic cells into the liquid bag, and adsorbing the immunomagnetic cells in the liquid bag through the magnetic platform;
controlling the magnetic platform to swing according to preset swing parameters to evenly distribute the immunomagnetic cells adsorbed by the magnetic platform, while allowing other substances in the liquid bag except for the adsorbed immunomagnetic cells to be suspended in the liquid;
performing a waste liquid discharge operation to allow the liquid in the liquid bag, except for the adsorbed immunomagnetic cells, to flow into a waste liquid bag;
performing a cleaning operation on remaining immunomagnetic cells in the liquid bag; and
after injecting a resuspension into the liquid bag and removing the liquid bag containing the immunomagnetic cells, confirming that a magnetic bead sorting process is complete.

2. The magnetic bead sorting method according to claim 1, wherein the placing an empty liquid bag on a magnetic platform, inputting a liquid containing immunomagnetic cells into the liquid bag, and adsorbing the immunomagnetic cells in the liquid bag through the magnetic platform comprises:
placing the empty liquid bag on the magnetic platform of a pressing device, wherein the pressing device comprises a covering mechanism arranged on the magnetic platform and a pressing mechanism connected to the covering mechanism;
inputting the liquid containing the immunomagnetic cells into the liquid bag; and
after the covering mechanism is closed, controlling the pressing mechanism to drive the closed covering mechanism to move downwards to flatten the liquid bag placed in an accommodating space between the magnetic platform and the covering mechanism, thereby increasing a contact surface between a bottom surface of the liquid bag and the magnetic platform, and then adsorbing the immunomagnetic cells in the liquid bag onto the contact surface through the magnetic platform.

3. The magnetic bead sorting method according to claim 2, wherein the pressing device further comprises a lifting mechanism connected to the covering mechanism;
after the inputting the liquid containing the immunomagnetic cells into the liquid bag, the magnetic bead storing method further comprises:
when the covering mechanism is pushed up by expanded liquid bag which is expanded and is incapable of being closed, controlling the lifting mechanism to drive the covering mechanism to ascend, thereby increasing the accommodating space between the magnetic platform and the covering mechanism for placing the liquid bag;
after the covering mechanism ascends to a preset height at which the covering mechanism is capable of being closed, closing the covering mechanism;
controlling the pressing mechanism to drive the closed covering mechanism to move downwards, thereby flattening the liquid bag placed in the accommodating space, increasing the contact surface between the bottom surface of the liquid bag and the magnetic platform, and thus adsorbing the immunomagnetic cells in the liquid bag onto the contact surface through the magnetic platform.

4. The magnetic bead sorting method according to claim 1, wherein the controlling the magnetic platform to swing according to preset swing parameters comprises:
obtaining preset mixing parameters comprising a mixing angle and a mixing duration;
controlling a mixing device to continuously swing the magnetic platform at the mixing angle for the mixing duration, such that the immunomagnetic cells are evenly adsorbed on the magnetic platform and the other substances, except for the adsorbed immunomagnetic cells, are suspended in the liquid as the magnetic platform swings.

5. The magnetic bead sorting method according to claim 4, wherein after the mixing device controls the magnetic platform to continuously swing at the mixing angle for the mixing duration, and before the performing a waste liquid discharge operation, the magnetic bead sorting method further comprises:
placing the magnetic platform horizontally for a preset duration;
obtaining first gentle swing parameters comprising a first gentle swing angle and a first gentle swing duration, wherein an absolute value of the first gentle swing angle is smaller than an absolute value of the mixing angle;
controlling the mixing device to continuously swing the magnetic platform at the first gentle swing angle for the first gentle swing duration, such that the other substances in the liquid bag, except for the adsorbed immunomagnetic cells, are suspended in the liquid.

6. The magnetic bead sorting method according to claim 4, wherein the mixing device comprises a first motor, a speed reducer, a first driving wheel, a first driven wheel, a first synchronous belt, and a synchronous bracket; the speed reducer is connected to an output shaft of the first motor to increase an output torque of the first motor while reducing a speed of the first motor; the first driving wheel is connected to a rotating shaft of the speed reducer, the first synchronous belt is sleeved on the first driving wheel and the first driven wheel, the first driven wheel is arranged on the magnetic platform, and the magnetic platform is rotatably arranged on the synchronous bracket;
the controlling a mixing device to continuously swing the magnetic platform at the mixing angle comprises:
determining operating parameters of the first motor based on the mixing angle;
turning on the first motor to drive the speed reducer with the operating parameters to drive the first driving wheel to rotate, driving the first driven wheel to rotate by the first driving wheel through the first synchronous belt, thereby driving the magnetic platform to swing around a connection point with the synchronous bracket.

7. The magnetic bead sorting method according to claim 1, wherein the performing a waste liquid discharge operation comprises:
controlling the magnetic platform to stay at a first preset staying angle, such that the liquid bag tilts downwards from a liquid inlet of the liquid bag towards a liquid outlet of the liquid bag;
performing a first switch operation to open a waste liquid discharge pipeline, wherein the waste liquid discharge pipeline comprises a pump and a bubble sensor connected between the waste liquid bag and the liquid outlet of the liquid bag;
discharging the liquid in the liquid bag, except for the adsorbed immunomagnetic cells, into the waste liquid bag through the waste liquid discharge pipeline; and
confirming the waste liquid discharge operation is complete when the bubble sensor determines that no liquid is passing through the waste liquid discharge pipeline.

8. The magnetic bead sorting method according to claim 7, wherein the performing a cleaning operation on remaining immunomagnetic cells in the liquid bag comprises:
controlling the magnetic platform to stay at a second preset staying angle, such that the liquid bag tilts upwards from the liquid inlet of the liquid bag towards the liquid outlet of the liquid bag, wherein a direction of the second preset staying angle is opposite to that of the first preset staying angle;
performing a second switch operation to open a cleaning pipeline, wherein the second switch operation comprises turning off the pump and a channel between the bubble sensor and the waste liquid bag, and connecting the pump and the bubble sensor between a cleaning solution liquid inlet and the liquid inlet of the liquid bag;
inputting a first preset volume of the cleaning solution into the liquid bag through the cleaning pipeline;
obtaining second gentle swing parameters comprising a second gentle swing angle and a second gentle swing duration;
controlling the mixing device to continuously swing the magnetic platform at the second gentle swing angle for the second gentle swing duration, and then performing the waste liquid discharge operation again.

9. The magnetic bead sorting method according to claim 7, wherein the injecting a resuspension into the liquid bag and removing the liquid bag containing the immunomagnetic cells comprises:
adjusting the magnetic platform to be parallel to a horizontal plane;
performing a third switch operation to open a resuspension input pipeline, wherein the third switch operation comprises turning off the pump and a channel between the bubble sensor and the waste liquid bag, and connecting the pump and the bubble sensor between a resuspension inlet and the liquid inlet of the liquid bag;
inputting a second preset volume of the resuspension into the liquid bag through a resuspension input pipeline;
performing a fourth switch operation comprising turning off the pump and the channel between the bubble sensor and the resuspension inlet, and controlling the pump and the bubble sensor to communicate with atmosphere through a sterile air filter;
when the bubble sensor determines that no liquid is passing through the channel between the bubble sensor and the resuspension inlet, it is determined that the resuspension is completely injected into the liquid bag; and
after performing thermal sealing on the liquid outlet and the liquid inlet of the liquid bag, removing the liquid bag containing the immunomagnetic cells from the magnetic platform.

10. The magnetic bead sorting method according to claim 9, wherein the inputting a second preset volume of the resuspension into the liquid bag through a resuspension input pipeline comprises:
injecting the resuspension into the liquid bag through the resuspension input pipeline;
obtaining weight change information of the liquid bag through a weighing sensor, and determining an actual volume of the injected resuspension based on the weight change information of the liquid bag; or, obtaining volume to rotation number data corresponding to the pump, and determining the actual volume of the injected resuspension based on an actual rotation number of the pump and the volume to rotation number data;
when the actual volume reaches the second preset volume, confirming that the second preset volume of the resuspension has been input into the liquid bag.

11. A magnetic bead sorting device comprising a pressing device, a mixing device, and a controller for performing the magnetic bead sorting method according to any one of claims 1 to 10, wherein the controller is connected to the pressing device and the mixing device.

12. A computer-readable storage medium storing computer-readable instructions, wherein the computer-readable instructions, when being executed by a processor, implement the magnetic bead sorting method according to any one of claims 1 to 10.
